# EUROPEAN PATENT APPLICATION

(11) **EP 1 085 004 A1**
(43) Date of publication of application: **21.03.2001**
(21) Application number: 99203061.9
(22) Date of filing: 20.09.1999
(51) Int. Cl.: C07C 51/14, C07C 57/03

(54) **Process for the carbonylation of butadiene and/or butadiene derivative**

(71) Applicant: DSM N.V., 6411 TE Heerlen (NL)
(72) Inventor: Sielcken, Otto Erik, 6136 BG Sittard (NL); Boogers, Jeroen Antonius Franciscus, 6224 JK Maastricht (NL); Smeets, Theodurus Maria, 6181 BP Stein (NL); Schaffrath, Heike, 52064 Aachen (DE); Baur, Henricus Anna Christiaan, 6049 BE Roermond (NL); Agterberg, Frank Petrus Willibrord, 6118 EH Nieuwstadt (NL)
(74) Representative: Kleiborn, Paul Erik

(57) **Abstract**

Process for the carbonylation of butadiene and/or at least one butadiene derivative in the presence of carbon monoxide, an alcohol and/or water, a rhodium-containing catalyst and an iodide containing promotor, wherein the carbonylation and/or carbonylation product recovery is performed in the presence of at least one stabilizer compound selected from a compound of an element of any of the groups 1-2 of the Periodic Table, a first row transition metal compound of any of the groups 3-12 of the Periodic Table, a compound of an element of group 13 of the Periodic Table, a compound of an element of group 15 of the Periodic Table, a rhenium compound, an iridium compound, a platinum compound and a cerium compound.

## Description

The invention relates to a process for the carbonylation of butadiene and/or at least one butadiene derivative in the presence of carbon monoxide, an alcohol and/or water, a rhodium-containing catalyst and an iodide containing promotor.

A carbonylation reaction according to this invention means every reaction between an unsaturated substrate and a hydroxy group containing reactant and carbon monoxide, in which an acid or ester compound is obtained.

Such a carbonylation reaction is described in EP-A-428979. This patent publication describes the preparation of 3-pentenoic acid by carbonylation of 2-buten-1-ol, 3-buten-2-ol or crotyl acetate using a rhodium carbonylation catalyst system promoted with HI.

A disadvantage of this process is that the separation of the rhodium catalyst and the carbonylation product 3-pentenoic acid is cumbersome because the rhodium catalyst tends to form solid and/or volatile rhodium compounds. The separation has to be performed in the presence of high amounts of carbon monoxide in order to prevent that solid rhodium compounds are formed.

The object of the invention is to provide a process for the preparation of pentenoic acid or ester thereof in which the rhodium catalyst and pentenoic acid or ester thereof can easily be separated.

This object is achieved in that the carbonylation and/or carbonylation product recovery is performed in the presence of at least one stabilizer compound selected from a compound of an element of any of the groups 1-2 of the Periodic Table, a first row transition metal compound of any of the groups 3-12 of the Periodic Table, a compound of an element of group 13 of the Periodic Table, a compound of an element of group 15 of the Periodic Table, a rhenium compound, an iridium compound, a platinum compound and a cerium compound.

It has now been found that with the process according to the invention the separation of pentenoic acid or pentenoate ester from the rhodium catalyst system can be performed under reduced pressure. The separation can for example easily be performed using vacuum distillation. The fact that in the process according to the invention the separation of pentenoic acid or pentenoate ester and the rhodium catalyst can be performed in a vacuum distillation unit can be regarded as a major advantage.

An additional advantage is that in the process according to the invention the formation of saturated dicarboxylic acids (ethylsuccinic acid, methylglutaric acid and adipic acid) and/or nonanoic acid is lowered. The formation of these high boiling compounds is disadvantageous because these high boiling compounds tend to accumulate in the recirculating catalyst stream. It is therefore necessary to separate these high boiling compounds by means of a purge.

The group 1 element of the Periodic Table can be any of the elements of group 1 of the Periodic Table as defined in Handbook of Chemistry and Physics, 70th edition, i.e Li, Na, K, Rb, Cs, Fr. A preferred group 1 element is Li.

The group 2 element of the Periodic Table can be any of the elements of group 2 of the Periodic Table as defined in Handbook of Chemistry and Physics, 70th edition, i.e Be, Mg, Ca, Sr, Ba and Ra. A preferred group 2 element is Mg.

The first row transition metal compound of any of the groups 3-12 of the Periodic Table, the group 13 element compound, the group 15 element compound, the rhenium compound, the iridium compound, the platinum compound and the cerium compound may comprise any compound which is soluble in the liquid reaction composition. The compound may be added to the liquid reaction composition for the carbonylation reaction in any suitable form which dissolves in the liquid reaction composition or is convertible to soluble form.

The first row transition metal of the groups 3-12 of the Periodic Table can be any of the metals designated as being in the first row of the groups 3-12 of the Periodic Table as defined in Handbook of Chemistry and Physics, 70th edition, i.e Sc, Ti, V, Cr, Mn, Fe, Co, Ni, Cu and Zn.

EP-A-749949 describes a process for carbonylating methyl acetate in the presence of a catalyst system containing rhodium, methyl iodide and a manganese stabiliser compound. This patent publication does not disclose or suggest the use of a manganese rhodium catalyst stabiliser in the carbonylation of butadiene or a derivative thereof.

The group 13 element of the Periodic Table can be any of the elements of group 13 of the Periodic Table as defined in Handbook of Chemistry and Physics, 70th edition, i.e B, Al, Ga, In, Tl. A preferred group 13 element is Ga.

The group 15 element of the Periodic Table can be any of the elements of group 15 of the Periodic Table as defined in Handbook of Chemistry and Physics, 70th edition, i.e N, P, As, Sb, Bi. Preferred group 15 elements are N and P.

It has been found that when the carbonylation according to the present invention is performed in the presence of a magnesium, vanadium, manganese, cobalt, zinc, gallium, rhenium, iridium and/or platinum compound the carbonylation reaction rate can be maintained at surprisingly high levels. This is advantageous because the higher the reaction rate, the smaller the required process equipment and/or the lower the required rhodium catalyst concentration. From an economical/investment point of view small process equipment and low rhodium concentration are desired.

Examples of these preferred compounds are vanadium, manganese, cobalt, zinc, gallium, rhenium, iridium or platinum acetate, acetyl acetonate, oxide or halogenide, or a carbonyl complex. Non-limitative examples thereof are MgCl₂, V(acac)₃, Mn(OAc)₂, Co(acac)₂, Zn(acac)₂, Ga(III)(acac)₃, Ga(III)(NO₃)₃, Ir(III)(acac)₃ and PtI₄ where acac is acetylacetonate and ac is acetate.

It has surprisingly been found that the presence of vanadium, manganese, zinc and/or iridium compound(s) in the carbonylation reaction of the present invention markedly enhances the selectivity towards 3-pentenoic acid or 3-pentenoate ester and the carbonylation reaction rate, as well as stabilizing the catalyst during the product separation stage.

It is believed that the iridium or platinum compound is active in the process according to the invention when it comprises iridium or platinum in a high oxidation state such as Ir(III) and/or Pt(IV). Thus, if the iridium or platinum is added to the reaction composition in a lower oxidation state, for example as Ir(I) or Pt(II), it may not exhibit the effect indicated above unless or until it is converted to a higher oxidation state, for example by contacting with a suitable oxidizing agent such as for example HI, I₂ and O₂.

In one preferred embodiment of the invention the carbonylation is performed in the presence of both an Ir compound and a Zn compound. It has been found that the concurrent use of a Ir and Zn compound results in a reduced formation of undesired by-products. An example of an undesired byproduct is valeric acid. Valeric acid can for example be formed by by carbonylation of butene and/or by hydrogenation of pentenoic acid. Hydrogenation may for example occur due the presence of hydrogen in the carbon monoxide and/or generation of hydrogen in situ by the water gas shift reaction.

When the carbonylation is performed in the presence of water as hydroxy group containing reactant, the main product will be pentenoic acid. In case an alcohol is used or formed during the reaction, as the hydroxy group containing reactant, a pentenoate ester will be formed as the main product.

The pentenoic acid will be obtained as a mixture of 2-, 3- and 4-pentenoic acid. The pentenoate ester will be obtained as a mixture of 2-, 3- and 4-pentenoate ester.

Non-limiting examples of alcohols include the group of C₁ to C₁₀ alkyl alcohols, including methanol, ethanol, 1-propanol, 2-propanol, the butanols, pentanols and hexanols.

The molar ratio of each stabilizer compound and rhodium lies generally between 1:10 and 100:1, preferably between 1:1 and 50:1 and more preferably between 1:1 and 10:1.

In the present application, the term butadiene derivatives means those compounds which yield pentenoate ester or pentenoic acid as the major product when carbonylated with the process according to the invention.

Suitable butadiene derivatives include the allylic butenols, allylic butenol esters, allylic butenyl ethers and/or allylic butenyl iodides.

The allylic butenol preferably is 3-butene-2-ol, 2-butene-1-ol or a mixture thereof.

Suitable allylic butenyl ethers and allylic butenol esters respectively are of the following formula (1): 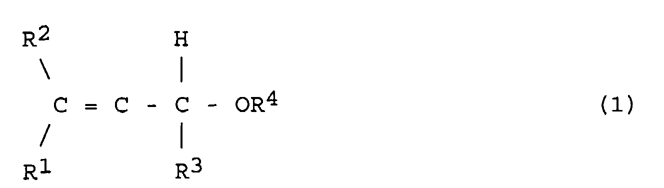 in which one of the groups R¹, R² and R³ is methyl and the other two groups are H and R⁴ is a C₁-C₁₀ alkyl group and 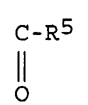 respectively. R⁵ is a C₁-C₁₀ alkyl group or alkenyl group. Non-limitative examples of allylic butenyl ethers are 1-methoxy-2-butene, 3-methoxy-1-butene, 1-ethoxy-2-butene, 3-ethoxy-1-butene. Non-limitative examples of allylic butenol esters are crotyl acetate, crotyl propionate, crotyl valerate, crotyl adipate, crotyl-2-pentenoate, crotyl-3-pentenoate and crotyl-4-pentenoate. Preferred allylic butenol esters are crotyl acetate, crotyl valerate, crotyl-2-pentenoate, crotyl-3-pentenoate and/or crotyl-4-pentenoate. Most preferred allylic butenol esters are crotyl acetate, crotyl-2-pentenoate, crotyl-3-pentenoate and/or crotyl-4-pentenoate.

For purpose of the present invention, mixtures that produce the allylic butenols, allylic butenyl ether or allylic butenol esters in situ are equivalent starting materials.

The allylic butenols can simply be prepared by hydrogenation of allylic butenal. The allylic butenols can also simply be prepared by reaction of butadiene with water as described in, for example, US-A-4645863. Esters of allylic butenols can be made by a method known for a man skilled in the art, for example, by reaction of the allylic butenols or of butadiene with the carboxylic acid 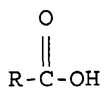 as described in, for example, US-A-4645863. Allylic butenyl ethers can also be made by a method known for a man skilled in the art, for example by reaction of butadiene with a C₁-C₁₀ alkyl alcohol as described in, for example, US-A-4343120 and US-A-4590300.

In one embodiment of the invention the carbonylation involves reacting butadiene, an allylic butenol and/or an allylic butenol ester and water to 3-pentenoic acid. In another embodiment of the invention an alkoxybutene is carbonylated to the corresponding alkyl-3-pentenoate. With an alkoxybutene is meant a compound according to formula (1) in which one of the groups R¹, R² and R³ is methyl and the other two groups are H and R⁴ is a C₁-C₁₀ alkyl group.

In another embodiment of the invention the carbonylation involves reacting butadiene and methanol to methyl-3-pentenoate.

The rhodium catalyst can be provided from any source or by any material which is soluble in the liquid reaction composition. Among the materials which can be employed as the source of the rhodium catalyst are rhodium metal, rhodium salts, rhodium oxides, rhodium carbonyl compounds, organorhodium compounds, coordination compounds of rhodium, and mixtures thereof. Specific examples of such materials include, but are not limited to, rhodium (III)chloride and its hydrates, RhI₃, Rh(CO)₂I₃, Rh(CO)I₃, rhodium(III)nitrate trihydrate, Rh₄(CO)₁₂, [Rh(CO)₂I]₂, Rh₆(CO)₁₆, Rh(acac)₃, Rh(CO)₂(acac), Rh(C₂H₄)₂(acac), Rh(CO)2 acetate, [Rh(C₂H₄)₂Cl]₂, [Rh(CO)₂Cl]₂, Rh(COD)(acac), [Rh(COD)Cl]₂, RhCl(CO)(PPh₃)₂, Rh₂[O₂C(CH₂)₆CH₃]₄ and Rh₂(acetate)₄, where acac is acetylacetonate, COD is 1,5-cyclooctadiene and Ph is phenyl. Examples of sources of rhodium catalyst include rhodium(I) compounds such as Rh(CO)₂(acac), [Rh(CO)₂I]₂, Rh(COD)(acac), and rhodium(III)iodide compounds such as RhI₃, Rh(CO)₂I₃. Supported rhodium compounds can also be used as a source of the rhodium catalyst. Examples of supported rhodium compounds are Rh/C, Rh/alumina and rhodium on polymeric supports, e.g. rhodium on polyvinylpyridine and rhodium on polyvinylpyrrolidon.

Suitable concentrations of rhodium in the reaction medium are in the range of 0.005-0.50 % by weight of rhodium metal based on the weight of the reaction medium. Preferably, the concentration of rhodium is in the range of 0.01-0.20 wt.%.

The rhodium, which may be pre-formed or generated in situ, must be promoted with the addition of an iodide containing promoter to achieve a satisfactory rate and selectivity to pentenoic acid and/or pentenoate ester. Examples of suitable iodide containing compounds are I₂, HI, organic iodide compounds and mixtures thereof. Examples of organic iodide compounds are acetyl iodide and lower alkyl (C₁-C₁₀) iodides. Examples of these alkyl iodides are methyl iodide, iodoethane, 2-iodobutane, 1,4-diiodobutane, 2-iodopropane, 1-iodopropane and iodoheptane. 2-Iodobutane is already reversible formed in the process from the reaction of butene by-products and HI and is therefore an especially good alkyl iodide source. When 3-pentenoic acid is the desired carbonylation product, it is advantageous to use an iodide containing compound. The most preferred compound is HI. When an alkyl-3-pentenoate is the desired carbonylation product, especially when the corresponding alkoxybutene is employed as the carbonylatable reactant, it is especially advantageous to use I₂ as iodine containing compound dissolved in an organic solvent, because the use of I₂ results in a reduced formation of 3-pentenoic acid compared with the use of for example HI. Suitable solvents for this embodiment of the invention include aromatic hydrocarbon solvent, saturated halocarbon solvents, and mixtures thereof. Carboxylic acid esters and lactones, such as methyl-3-pentenoate, valerolactone and the like, are also acceptable solvents. Suitable aromatic hydrocarbon solvents include chlorinated and fluorinated hydrocarbons such as methylene chloride, dichloroethane and cloroform as well as the so-called HCFC's including in particular HCFC-113 (FCCI₂CF₂CI) and HCFC-123 (CHCl₂CF₃) or the like. The most preferred solvents are toluene, HCFC-113 and HCFC-123.

Generally, the concentration of (inorganic) iodide in the reaction medium is at least 0.05 wt.%. The initial concentration of iodide in the reaction medium is generally lower than 2 wt.%.

The temperature of the reaction is generally between 40°C and 250°C, but best rates and yields are achieved between 100°C and 160°C. Above 160°C, yields to pentenoic acid are reduced. Below 100°C, reaction rates become too slow for practical commercial exploitation.

The carbon monoxide partial pressure applied during the carbonylation reaction is in the range between 0.5 and 20 MPa, preferably between 1.5 and 15 MPa.

The carbonylation reaction of butadiene or butadiene derivatives are generally performed in a solvent.

Suitable solvents are aliphatic and aromatic carboxylic acid solvents, aromatic hydrocarbon solvents, and mixtures thereof. More specifically, suitable carboxylic acids include aliphatic C₂-C₂₀ monocarboxylic acids, aliphatic C₄-C₂₀ dicarboxylic acids, benzoic acid, C₇-C₁₀ alkyl-substituted benzoic acids, and esters and/or ethers thereof and mixtures thereof. Suitable aromatic hydrocarbon solvents include benzene and C₁-C₄ alkyl substituted benzenes. The reactants may also serve as solvent. The preferred solvents are benzene, toluene, aliphatic C₂-C₁₀ monocarboxylic acids, C₄-C₇ dicarboxylic acids, benzoic acid and mixtures thereof. The most preferred solvents are acetic, propionic, butyric, 3-pentenoic acid, 4-pentenoic acid, 2-methylbutyric, valeric, and caproic acids, benzene, toluene, and mixtures thereof. Mixtures of monocarboxylic and dicarboxylic acids can also be used in whole or in part as the solvent for this process. Such monocarboxylic and dicarboxylic acids include nonanoic, adipic, valeric, 2-methylglutaric, 3-methylglutaric, ethylsuccinic and methylbutyric acids. The preferred solvent for a commercial process is the mixture of acid byproducts that are less volatile than pentenoic acid or pentenoate ester. These are mostly six carbon diacids (for example adipic acid, 2-methylglutaric acid and 2-ethylsuccinic acid) and saturated and unsaturated nine carbon acids (for example nonanoic acid and cyclohexylpropionic acid).

Pentenoic acid and the ester thereof are important intermediates in a process to prepare adipic acid (precursor for Nylon-6.6) or ε-caprolactam (precursor for Nylon-6).

The pentenoic acid or pentenoate ester carbonylation product may be recovered from the liquid reaction medium by withdrawing liquid reaction medium from the reactor and separating the carbonylation product by one or more flash and/or fractional distillation stages from the other components of the liquid reaction medium such as for example rhodium catalyst, iodide promotor and the stabilizing compound which fraction is preferably recycled to the reactor.

The process according to the present invention may be operated in a batch, semi-continuous or continuous mode, preferably in a continuous mode.

The continuous process is preferably performed by continuously removing part of the liquid reaction medium from the carbonylation reactor. This mixture comprises the pentenoic acid and/or an ester thereof, the rhodium catalyst, the iodide containing promotor, the stabilizing compound, optionally the solvent, by-products, unreacted butadiene or butadiene derivative and carbon monoxide dissolved in said medium. In a first step carbon monoxide is removed from this mixture by reducing the pressure in for example a flash operation. This carbon monoxide may be reused in the carbonylation. The 3-pentenoic acid and/or 3-pentenoate ester, unreacted butadiene or butadiene derivative and low boiling by-products, for example acetic acid in case crotyl acetate is carbonylated, can advantageously be recovered from the rhodium catalyst, the stabilizer compound and high boiling compounds, for example six carbon diacids, by vaporisation of the liquid reaction mixture at a partial pressure of carbon monoxide which is substantial less than in the carbonylation reactor. From an economical point of view the vaporisation is preferably performed at a carbon monoxide partial pressure lower than 0.1 MPa, more preferably the vaporisation is preferably performed in the absence of carbon monoxide. From the point of view of catalyst stability the carbon monoxide partial pressure is preferably higher than 1 kPa, more preferably higher than 10 kPa. High temperatures will increase the vapor pressure of pentenoic acid or pentenoate ester and make the separation more efficient, but will also destabilise the rhodium more rapidly. From an economical point of view the vaporisation is preferably performed at a temperature near the atmospheric boiling points of pentenoic acid or pentenoate ester. From the point of view of catalyst stability the vaporisation is preferably performed at a temperature lower than 140°C, more preferably lower than 135°C. It has been found that when the recovery of the rhodium catalyst from the liquid reaction mixture is performed at a carbon monoxide partial pressure of between 1 kPa and 0.1 MPa and a temperature between 100°C and the atmosphere boiling point of pentenoic acid or pentenoate ester, the rhodium catalyst can easily be recovered from the reaction mixture using only one flash distillation stage. The fact that the rhodium catalyst can easily be recovered from the reaction mixture in only one flash distillation stage can be regarded as a major advantage. Subsequently the rhodium catalyst containing fraction is preferably recycled to the carbonylation reactor.

The invention will be further eludicated by means of the following, non-limiting examples.

The selectivity and productivity described in the examples and experiments are defined in the following manner:

The selectivity to 3-pentenoic acid or 3-pentenoate ester is the amount of substrate compound which has been converted to 3-pentenoic acid or 3-pentenoate ester divided by the amount of substrate compound which has been reacted.

The productivity is the obtained amount (mol) of 3-pentenoic acid or 3-pentenoate ester per liter reaction medium per hour.

### Example I-XI

A 160 ml Parr mechanically stirred Hastelloy-C autoclave was charged with ± 500 ppm Rh(CO)₂acac, stabilizing compound (see Table I), ± 5 wt.% water, and 50 ml of acetic acid.

The solution was heated to a temperature of 130°C with agitation (1000 rpm) under an initial pressure of 1 MPa carbon monoxide. The reaction was initiated by injection of ± 10 wt.% crotyl alcohol (CrOH) (see Table 1) and adjusting the total pressure to 5 MPa. The exact amounts are given in Table 1. During the run the total pressure was kept between 3 and 5 MPa by feeding carbon monoxide from a reservoir at intervals.

Samples were taken at intervals and analyzed using GC techniques.

Catalyst stability was tested in the following way. The reaction mixture was cooled to ambient temperature at 5 MPa CO and taken to a thin film evaporator and/or a short path (wiped) film evaporator for distillation under vacuum at 122°C wall temperature and 13 kPa pressure, feed rate of 5 ml/min and atmospheric CO shielding (10 Nl/h). The liquid hold-up time was estimated at 3-5 minutes. The visual observation of Rh precipitate was taken as a criterion for catalyst stability.

The results are presented in Table 1.

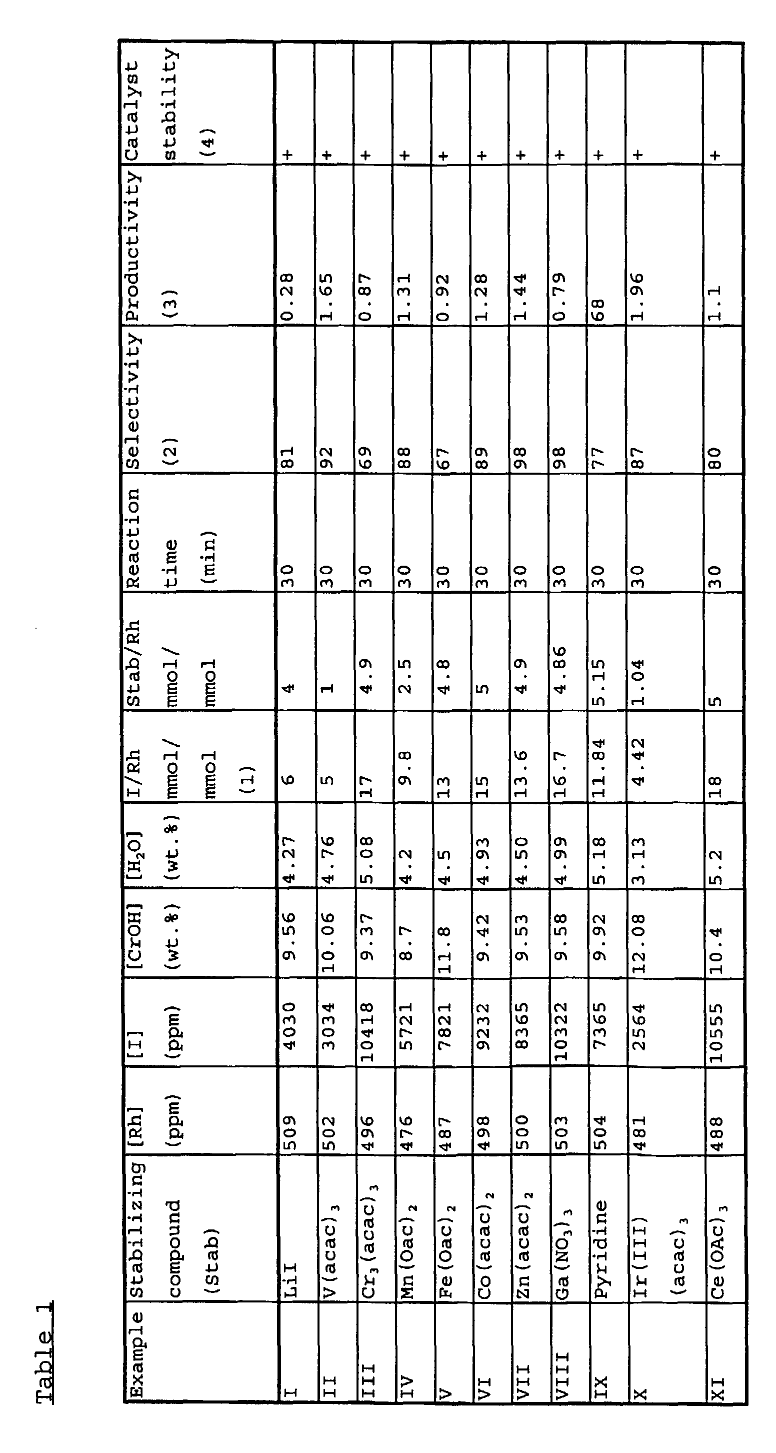

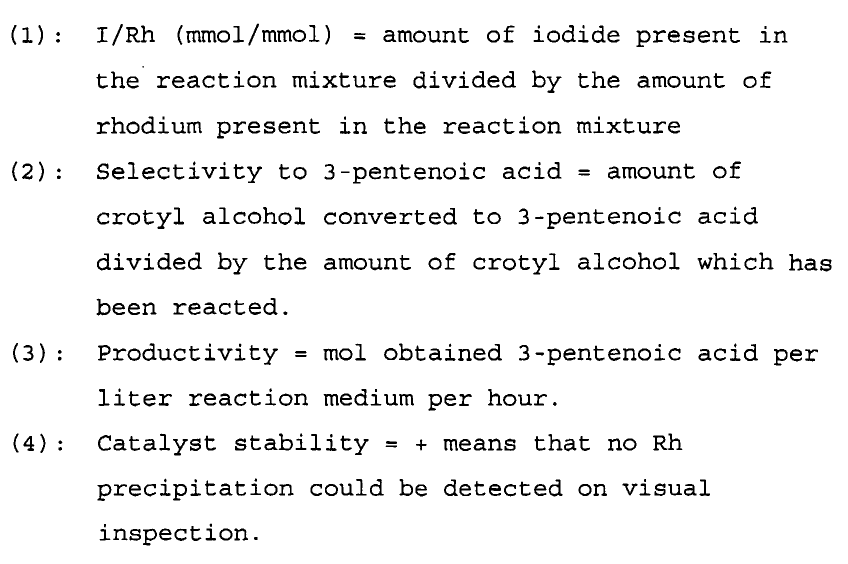

### Example XII

A 160 ml Parr mechanically stirred Hastelloy-C autoclave was charged with 0.24 mmol Rh(acac)(CO)₂, 0.56 mmol Ir(acac)₃, 1.70 mmol HI, 671 mmol acetic acid and 106 mmol H₂O (Ir/Rh = 2.3) and I/Rh = 7.1.
The solution was heated to a temperature of 130°C with agitation (1000 rpm) under an initial pressure of 1 MPa carbon monoxide. The reaction was initiated by injection of 72 mmol crotyl alcohol and adjusting the total pressure to 5 MPa. During the run the total pressure was kept between 3 and 5 MPa by feeding carbon monoxide from a reservoir at intervals.

After one hour 10 mmol 3-pentenoic acid, 4 mmol 2-pentenoic acid and 15 mmol valeric acid have been formed (48 mol% pentenoic acid and 52 mol% valeric acid).

### Example XIII

Example XII was repeated with 0.274 mmol Rh(acac)(CO)₂, 0.55 mmol Ir(acac)₃ (Ir/Rh = 2), 1.24 mmol Zn(acac)₂ (Zn/Rh = 4.5), 3.17 mmol HI (I/Rh = 11.6), 770 mmol acetic acid and 109 mmol H₂O and 75 mmol crotylalcohol.

After one hour 43 mmol 3-pentenoic acid, 1 mmol crotyl-3-pentenoate and 0.2 mmol valeric acid have been formed (0.5 mol% valeric acid and 99.5 mol% pentenoic acid/pentenoate ester).

### Comparative Experiment A

Example XII was repeated, except that no Ir(acac)₃ was added. The catalyst stability was tested as in Example I-XI. After only a few seconds Rh precipitate was detected.

### Comparative Experiment B

Rh/I catalyzed hydrocarboxylation followed by atmospheric CO stripping of 3-pentenoic acid product.

### Step 1: Reaction:

Catalyst solution was prepared with the following recipe: 1436.0 grams nonaoic acid, 2.2 grams rhodium dicarbonyl acetyl acetoate (acac), and 3.76 grams aqueous (54%) hydroiodic acid (HI). The mixture was stirred and sparged with carbon monoxide until dissolved. Half of this solution was charged to a 1 liter Hastelloy C276 autoclave which is equipped with a stirrer. The solution was stirred at 1500 rpm and heated rapidly to 140°C liquid temperature, under 2.4 MPa carbon monoxide pressure. When temperature was reached, liquid feed was introduced to the reactor from 2 syringe pumps, 1 feeding 1,3 butadiene at 50 ml/hr, the other feeding water at 19 ml/hr (slight excess water). Feed was continued until 1 hour had elapsed, approximately 5% by weight butadiene had been fed. The reactor was pressured to 3.4 MPa during the feed hour and left open to a regulated CO supply with uptake reader. The batch reaction was continued until uptake slowed to less than 20 SCCM. The reactor was then cooled and vented of CO pressure and the liquid drained into a product bottle. The second half of the catalyst mixture was then charged to the autoclave and the above reaction sequence was repeated to yield a total of nearly 1700 ml product solution consisting of over 90 wt% nonanoic acid, with 7.5 wt% 3-pentenoic acid and unreacted butadiene and water each less than 1 wt.%.

### Step 2: Product Recovery

300 grams of 3-pentenoic acid is added to the product solution from step 1 above to facilitate an easier separation via distillation. The resulting feed solution for stripping is 23.5 wt.% 3-pentenoic acid, 76.0 wt.% nonanoic acid, and 0.5 wt.% water. The stripper column is a glass 25 mm diameter Oldershaw type column with 35 plates, with the feed introduced 5 plates from the bottom (i.e. 30 plates of rectification). The column is jacketed with boiling Mesitylene under slight vacuum (162°C) for heating the liquid on all 35 trays. Carbon monoxide is fed to the column at 0.1 MPa, 10.0 SLPM, as a stripping gas. The use of carbon monoxide helps control temperatures in the column and stabilizes the rhodium catalyst for precipitation. The feed solution is fed to the column at 8.7 grams per minute with a rhodium concentration of 414 ppm, and an iodide concentration of 907 ppm. The liquid temperature in the column varied on each plate between 135 and 155°C. The large majority of the overhead material, after condensing, was returned to the top of the column as reflux. The ratio of liquid fed to the top of the column to distillate taken off the top of the column was 15:1. The overhead taken off at 1.12 grams per minute at a composition of 99.5 wt.% 3-pentenoic acid, 0.5 wt.% water, 0.30 ppm Rh and 441 ppm iodide. The large number of plates and high reflux ratio are necessary for near complete removal of rhodium from the overhead stream. The bottoms composition was 11.5 wt.% 3-pentenoic acid, with the remainder primarily nonanoic acid. The bottoms rhodium and iodide levels were 508 and 882 ppm respectively.

### Example XIV

### Carbonylation of butadiene with Rh/LiI promoted by HI

A 150 ml mechanically stirred Hastelloy-C autoclave was charged with 0.54 mmoles of Rh(acac)(CO)₂, 3.8 mmoles HI (added as 57 wt.% in water), 1.08 mmoles anhydrous LiI, 217 mmoles H₂O and 800 mmoles acetic acid. The solution was heated to a temperature of 130°C with agitation (1200 rpm) under an initial pressure of 1 MPa carbon monoxide. The reaction was initiated by injection of 106 mmoles of butadiene and adjusting the total pressure to 5 MPa. During the run the total pressure was kept between 4 and 5 MPa by feeding carbon monoxide from a reservoir at intervals.

Samples were taken at intervals and analyzed using different GC techniques for butadiene, monocarboxylic acids and dicarboxylic acids.

After 60 min, the selectivity to cis-and trans pentenoic acid was 79%, the selectivity to nonanoic acid was 0%, the selectivity to dibasic acids was 0%.

After 210 min, the selectivity to cis-and trans pentenoic acid was 78%, the selectivity to nonanoic acid was 0%, the selectivity to dibasic acids was 0%.

### Comparative Experiment C

Example XIV was repeated with the following amounts: 0.45 mmol (730 ppm) of Rh(acac)(CO)₂, 1.25 mmole of LiI, 324 mmoles of water, 50 grams of acetic acid and 93 mmoles of butadiene. No HI was added to the reaction mixture. The reaction was allowed to run for a total of 6 hours. The GC analysis indicated that the yield to 3-pentenoic acids was less than 4%.

### Comparative Experiment D

The experiment of Comparative Experiment C was repeated, except that an additional amount of 15 mmoles of anhydrous lithiumiodide was added. The GC analysis indicated that no 3-pentenoic acid was formed.

The results of Example XIV and Comparative Experiments C and D indicate that the rhodium-catalyzed conversion of butadiene to pentenoic acids is (completely) inhibited in the presence of only LiI (C) or a large excess of LiI (D), but works in the presence of LiI and HI with an excess of HI (XIV).

### Comparative Experiment E

### Carbonylation of butadiene with Rh/HI

A 150 ml mechanically stirred Hastelloy-C autoclave was charged with 0.45 mmole (730 ppm) of Rh(acac)(CO)₂, 1.25 mmoles of HI (added as 57 wt% HI in water), 324 mmoles of water, and 50 grams of acetic acid. The solution was heated to a temperature of 130° C with agitation (1200 rpm) under an initial pressure of 2 MPa carbon monoxide. The reaction was initiated by injection of 93 mmoles of butadiene and adjusting the total pressure to 5 MPa. During the run the total pressure was kept between 4 and 5 MPa by feeding carbon monoxide from a reservoir at intervals. Samples were taken at intervals and analyzed using different GC techniques for butadiene, monocarboxylic acids and dicarboxylic acids.

After one hour the selectivity to cis- and trans pentenoic acid was 91%, the selectivity to nonanoic acids was 2.7%, and the selectivity to dibasic acids (ethyl succinic acid, methyl glutaric acid, and adipic acid) was 0.9 %.
After 3 hours the selectivity to cis- and trans pentenoic acid was 86%, the selectivity to nonanoic acids was 4.9%, and the selectivity to dibasic acids (ethyl succinic acid, methyl glutaric acid, and adipic acid) was 6.2 %.

### Example XV

### Carbonylation of crotyl alcohol with Rh/LiI

A 150 ml mechanically stirred Hastelloy-C autoclave was charged with 0.68 mmoles of Rh(acac)(CO)₂, 20.27 mmoles of LiI, 239 mmoles of water, and 94.5 grams of valeric acid. The solution was heated to a temperature of 130°C with agitation (1000 rpm) under an initial pressure of 2 MPa carbon monoxide. The reaction was initiated by injection of 148 mmoles of crotyl alcohol and adjusting the total pressure to 5 MPa. During the run the total pressure was kept between 4 and 5 MPa by feeding carbon monoxide from a reservoir at intervals.

Samples were taken at intervals and analyzed using GC techniques.

After 1 hour of operation the selectivity to cis- and trans pentenoic acid and esters was 87%, the selectivity to nonanoic acids was 0%, and the selectivity to dibasic acids (ethyl succinic acid, methyl glutaric acid, and adipic acid) was 0%. Thereafter, the reaction mixture was concentrated by a factor 2, using a falling film evaporator at 122°C, 13 kPa and a small inlet of CO (10 l CO/hour). No Rh precipitation could be detected on visual inspection.

### Comparative Experiment F

The above experiment was repeated using HI instead of LiI. After 1 hour of operation the selectivity to cis- and trans pentenoic acid and esters was 50%, the selectivity to nonanoic acids was 0%, and the selectivity to dibasic acids (ethyl succinic acid, methyl glutaric acid, and adipic acid) was 25%. During the above concentration most of the Rh precipitated.

### Example XVI

### Carbonylation of butadiene with Rh/Mn promoted by HI

A 150 ml mechanically stirred Hastelloy-C autoclave was charged with 0.5 mmoles (900 ppm) of Rh(acac)(CO)₂, 1.1 mmoles Mn(OAc)₂, 4.7 mmoles HI (added as 57% HI in water), 200 mmoles water, and 50 grams of acetic acid. The solution was heated to a temperature of 130°C with agitation (1000 rpm) under an initial pressure of 1 MPa carbon monoxide. The reaction was initiated by injection of 100 mmoles of butadiene and adjusting the total pressure to 5 MPa. During the run the total pressure was kept between 4 and 5 MPa by feeding carbon monoxide from a reservoir at intervals.

Samples were taken at intervals and analyzed using different GC techniques for butadiene, monocarboxylic acids and dicarboxylic acids.

After 60 min, the selectivity to cis-and trans pentenoic acid was 81%, the selectivity to nonanoic acid 0%, the selectivity dibasic acids was 0%. The productivity was 0.65 mol/(l/h)

After 105 min, the selectivity to cis-and trans pentenoic was 81%, the selectivity to nonanoic acid 0%, the selectivity to dibasic acids was 0%.

The catalyst stability was tested as in Example I-XI. No Rh precipitation could be detected on visual inspection.

### Example XVII

### Carbonylation of butadiene with Rh/V promoted by HI

A 150 ml mechanically stirred Hastelloy-C autoclave was charged with 0.5 mmoles (780 ppm) of Rh(acac)(CO)₂, 0.75 mmoles V(acac)₃, 3.2 mmoles HI (added as 57% HI in water), 262 mmoles water, and 53 grams of acetic acid. The solution was heated to a temperature of 130°C with agitation (1000 rpm) under an initial pressure of 1 MPa carbon monoxide. The reaction was initiated by injection of 167 mmoles of butadiene and adjusting the total pressure to 5 MPa. During the run the total pressure was kept between 4 and 5 MPa by feeding carbon monoxide from a reservoir at intervals.

Samples were taken at intervals and analyzed using different GC techniques for butadiene, monocarboxylic acids and dicarboxylic acids.

After 60 min, the selectivity to cis-and trans-3-pentenoic acid was 85%, the selectivity to nonanoic acid 0%, the selectivity to dibasic acids was 0%. The productivity was 1.1 mol/(l.h). The catalyst stability was tested as in Example I-XI. No Rh precipitation could be detected on visual inspection.

The results of Examples XIV-XVII and Comparative Experiments E-F indicate that in the process according to the invention the amount of saturated dicarboxylic acids is lowered.

## Claims

1. Process for the carbonylation of butadiene and/or at least one butadiene derivative in the presence of carbon monoxide, an alcohol and/or water, a rhodium-containing catalyst and an iodide containing promotor, characterized in that, the carbonylation and/or carbonylation product recovery is performed in the presence of at least one stabilizer compound selected from a compound of an element of any of the groups 1-2 of the Periodic Table, a first row transition metal compound of any of the groups 3-12 of the Periodic Table, a compound of an element of group 13 of the Periodic Table, a compound of an element of group 15 of the Periodic Table, a rhenium compound, an iridium compound, a platinum compound and a cerium compound.

2. Process according to claim 1, characterized in that the molar ratio of each stabilizer compound and rhodium lies between 1:1 and 50:1

3. Process according to claim 1, characterized in that the carbonylation is performed in the presence of a magnesium, vanadium, manganese, cobalt, zinc, gallium, rhenium, iridium and/or platinum compound.

4. Process according to claim 3, characterized in that the carbonylation is performed in the presence of a vanadium, manganese, zinc and/or iridium compound(s).

5. Process according to any one of claims 1-4, characterized in that, the iridium compound is added to the carbonylation reaction mixture as iridium(III) compound.

6. Process according to claim 5, characterized in that, a zinc compound is also present in the carbonylation reaction mixture.

7. Process according to any one of claims 1-4, characterized in that, the platinum compound is added to the carbonylation reaction mixture as platinum (IV) compound.

8. Process according to any one of claims 1-7, characterized in that, the carbonylation is conducted starting from allylic butenol, allylic butenol ester, allylic butenyl ether and/or allylic butenyl iodide.

9. Process according to any one of claims 1-8, characterized in that, the carbonylation is conducted starting from an allylic butenyl ether according to formula (1) : 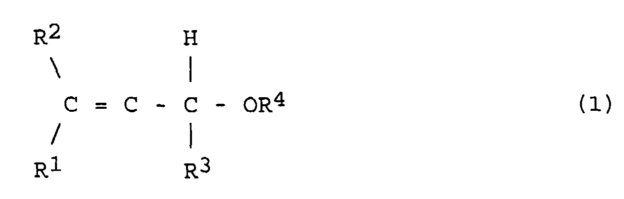 in which one of the groups R¹, R² and R³ is methyl and the other two groups are H and R⁴ is a C₁-C₁₀ alkyl group, and the iodide containing promotor is I₂ dissolved in an organic solvent.

10. Process according to any one of claims 1-9, characterized in that, the process is performed by continuously removing part of the liquid reaction medium, from the carbonylation reactor, comprising pentenoic acid and/or an ester thereof, the rhodium catalyst, the iodide containing promotor, the stabilizing compound, optionally the solvent, by-products, unreacted butadiene or butadiene derivative and carbon monoxide dissolved in said medium, removing carbon monoxide from this mixture by reducing the pressure, separating pentenoic acid and/or pentenoate ester, unreacted butadiene or butadiene derivative and low boiling by-products from the rhodium catalyst, the stabilizer compound, high boiling compounds and the optional solvent by vaporisation of the liquid reaction mixture at a partial pressure of carbon monoxide which is substantial less than in the carbonylation reactor and recycling the rhodium catalyst containing fraction to the carbonylation reactor.

11. Process according to claim 10, characterized in that the vaporisation is performed in one flash distillation stage at a carbon monoxide partial pressure of between 1 kPa and 0.1 MPa and a temperature between 100°C and the atmospheric boiling point of pentenoic acid or pentenoate ester.

12. Process as substantially described in the description and the examples.
